Europäisches Patentamt

European Patent Office

Office européen des brevets

(11) Publication number: **0 116 311**
**B1**

# EUROPEAN PATENT SPECIFICATION

(45) Date of publication of patent specification: **14.06.89**

(21) Application number: **84100398.1**

(22) Date of filing: **16.01.84**

(51) Int. Cl.⁴: **A 61 K 9/48, A 23 P 1/00,
B 01 J 13/02**

(54) **Double soft capsules and production thereof.**

(30) Priority: **17.01.83 JP 6287/83**

(43) Date of publication of application:
**22.08.84 Bulletin 84/34**

(45) Publication of the grant of the patent:
. **14.06.89 Bulletin 89/24**

(84) Designated Contracting States:
**AT BE CH DE FR GB IT LI LU NL SE**

(56) References cited:
**DE-A-2 729 068
DE-A-3 030 801
GB-A- 715 879
GB-A-1 538 510
GB-A-1 564 452**

(73) Proprietor: **MORISHITA JINTAN CO., LTD.
1-30, Tamatsukuri, 1-chome Higashi-ku
Osaka-shi Osaka-fu (JP)**
⑭ **AT BE CH DE FR GB IT LI LU NL SE**

(73) Proprietor: **FUJISAWA PHARMACEUTICAL CO.,
LTD.
3, Doshomachi 4-chome Higashi-ku
Osaka-shi Osaka 541 (JP)**
⑭ **BE CH LI LU NL SE AT**

(72) Inventor: **Morishita, Takashi
1-80, Kurakuen 5-bancho
Nishinomiya-shi Hyogo-ken (JP)**
Inventor: **Hata, Takehisa
13-7, Nishikakiuchi Terado-cho
Muko-shi Kyoto-fu (JP)**
Inventor: **Mori, Mitio
3-2-15, Shinikejima-cho
Higashiosaka-shi Osaka-fu (JP)**
Inventor: **Tanoue, Shohachi
10-23, Tokuranishi, 2-chome
Toyonaka-shi Osaka-fu (JP)**

(74) Representative: **Bühling, Gerhard, Dipl.-Chem.
et al
Patentanwaltsbüro Tiedtke-Bühling-Kinne
Grupe-Pellmann-Grams-Struif Bavariaring 4
D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Various soft capsules containing a medicine or a food stuff are widely on the market, and as those containing solid fillers therein, there are known oily soft capsules in which solid and oil agents are encapsulated with a capsule film-forming substance consisting mainly of gelatin (refer to Japanese Patent Publication No. 42044/1982).

DE—A—27 29 068 discloses a multiple capsule comprising as outer capsule a hard capsule made of hard gelatine. The capsule of hard gelatine can contain one or more capsules of soft gelatine. Capsules of soft gelatine can be used in connection with aqueous and oily suspensions or emulsions as filling. With the known multiple capsules it is possible to release the active substance of a medicine in a controlled manner.

DE—A—30 30 801 discloses a process for preparing a double capsule containing microcapsules embedded in a hydrophobic substance. The microcapsules can contain a hydrophilic or hydrophobic substance and the first mentioned hydrophobic substance should have a special viscosity of 50 to 150.000 mPa.s at 25°C. As film-forming substance of the double capsule is mentioned a urea/formaldehyde-polymer, a melamine/formaldehyde-polymer or a methylolmelamine/formaldehyde-polymer. The microcapsules have an average particle size of about 2 to 3 μm and the double capsules have a particle size of 2 to 10 μm. The known microcapsules are used for a pressure-sensitive recording paper.

GB—A—15 64 452 discloses a process for preparing encapsulated drops of fruit material wherein an aqueous fruit material is treated with an aqueous alginate or pectate sol and subsequently the coating being gelatinized with calcium or aluminium ions. This prior art is directed to only a production of a single soft capsule.

GB—A—1 538 510 discloses a method of manufacturing capsules, each consisting of a quantity of capsule filler material enclosed in one or more seamless films, the method comprising extruding the filler and film materials from the orifices to form the jet stream in a liquid cooling medium in the container, vibrating the annular vibratory body at a predetermined frequency to induce waves in the liquid cooling medium so that the jet stream is oscillated and is constricted to continuously form spherical capsules, and cooling the capsules in the liquid cooling medium to solidify the film material.

The object of the present invention is to provide a double soft capsule in which an inner soft capsule is contained with outer capsule liquid in an outer soft capsule, so that the use of soft encapsulated products is markedly improved.

The present invention provides double soft capsules containing an inner soft capsule with an outer capsule liquid in an outer soft capsule, the film-forming substance of the inner and outer capsule comprising (a) gelatine and (b) a water-soluble polyhydric alcohol or its water-soluble derivative.

Such double soft capsules are especially useful in combining two or more components which cannot be enclosed together in a single capsule or in the regulation of the rate of dissolution in vivo of the medicines by properly selecting the kind of the film-forming substance for the outer and inner soft capsules.

According to the invention the film-forming substance of the inner and outer capsule comprises (a) gelatin, and (b) a water-soluble polyhydric alcohol or its water-soluble derivative as an essential component. Further the film-forming substance is obtained by treating a composition containing (a) gelatin, (b) a water-soluble polyhydric alcohol or its water-soluble derivative and (c) a lower methoxylpectin having a molecular weight of not less than 200 000 and a degree of methoxylation of 1 to 6% or sodium alginate with a salt of a polyvalent metal to gelatinize an aqueous solution of said lower methoxylpectin or sodium alginate.

Gelatin and the water-soluble polyhydric alcohol or its water-soluble derivative, can be used in a grade so far used for producing soft capsules as such.

As water-soluble polyhydric alcohol or its water-soluble derivative, can be used, for example, glycerin, polyglycerin, sorbitol, ethylene glycol, polyethylene glycol, propylene glycol, polypropylene glycol, ethylene oxide/propylene oxide copolymer, and glyceride.

When gelatin and the water-soluble polyhydric alcohol or its water-soluble derivative are used as a film-forming substance for soft capsule, it is preferred that the amount of gelatin used and that of the water-soluble polyhydric alcohol or its water-soluble derivative used are 60 to 90 weight % and 10 to 40 weight %, respectively, based on the total weight of the capsule film.

When gelatin, a lower methoxylpectin and the water-soluble polyhydric alcohol or its water-soluble derivative are used as a film-forming substance for soft capsule, it is preferred that the amounts of the first, second and the latter used are 65 to 75 weight %, 5 to 20 weight %, preferably 10 to 15 weight %, and 10 to 30 weight %, respectively, based on the total weight of the capsule film.

When gelatin, sodium alginate and the water-soluble polyhydric alcohol or its water-soluble derivative are used as a film-forming substance for soft capsule, it is preferred that the amounts of the first, second and the latter used are 65 to 85 weight %, 1 to 10 weight %, and 10 to 30 weight %, respectively, based on the total weight of the capsule film.

A preferred lower methoxylpectin is that in which the degree of methoxylation is 3.5 to 5%.

To gelatinize aqueous solutions of said lower methoxylpectin or sodium alginate, there may be used the salt of polyvalent metals having two or more valencies, particularly the water-soluble salts of calcium and magnesium for a lower methoxylpectin, and water-soluble calcium salts such as calcium chloride and calcium phosphate for sodium alginate.

The double soft capsule according to the present invention may be used, in addition to those which are taken orally such as a medicine, or a foodstuff, for those which are applied to industrial uses such as various industrial products (e.g. two-component type adhesives, feeds, agricultural and horticultural chemicals, cosmetics and medical supplies. Consequently, the film-forming substance for the inner and outer soft capsules, kind of liquid to be encapsulated in the inner soft capsule (the liquid is shortened as an inner capsule liquid hereinafter) and size of the capsules, may properly be selected according to the object of use and usages.

The double soft capsule according to the present invention is suitable particularly in the field of medicine.

When the double soft capsule is a medicine, the rate of dissolution in vivo of the medicine can be regulated by properly selecting the kind of the film-forming substance for the outer and inner soft capsule.

For example, by making the film of the outer soft capsule soluble in the stomach by preparing the film from the foregoing film-forming components (a) and (b), and by making the film of the inner soft capsule soluble in the intestine by preparing the film from the foregoing film-forming components (a), (b) and (c), various excellent effects can be obtained, for example, the efficacy of medicines can be maintained in vivo, or medicines can be protected from decomposition by gastric juice.

Next, production of the double soft capsule according to the present invention will be illustrated with reference to the accompanying drawings.

Figs. 1 and 2 are schematic vertical sectional views illustrating the embodiments of nozzle part for producing a double soft capsule.

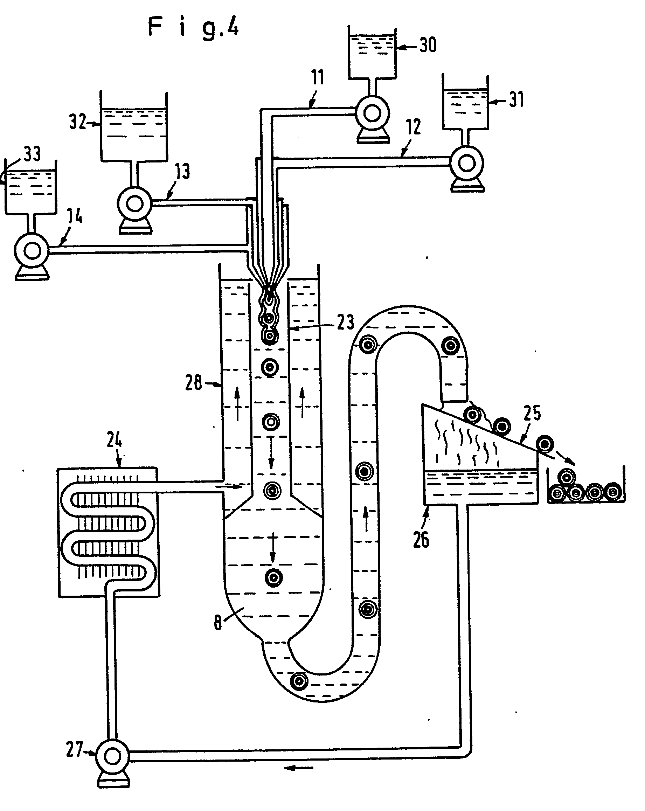

Figs. 3 and 4 are schematic flow sheets illustrating the production of a double soft capsule.

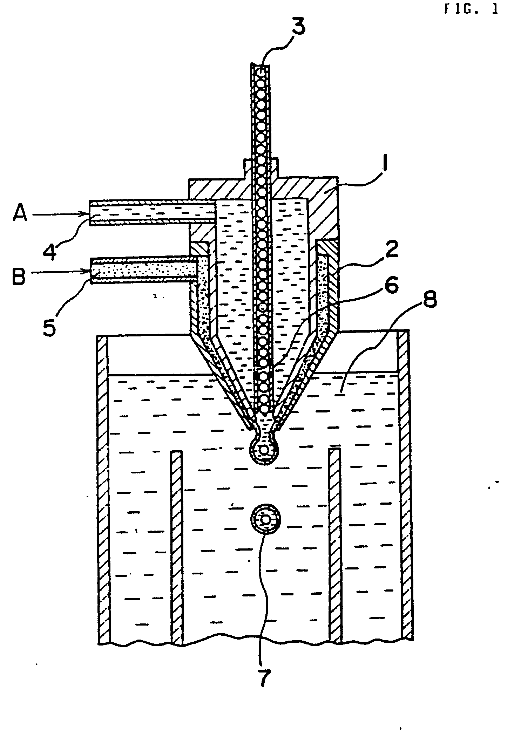

Fig. 1 is a schematic vertical sectional view illustrating one embodiment of the nozzle part of a suitable equipment for producing the double soft capsule according to the present invention.

The double soft capsule (7) according to the present invention is obtained as follows:

An inner soft capsule (3) (containing a proper inner capsule liquid) sent to the nozzle part, and an outer capsule liquid (4) to be filled in an outer soft capsule, as sent from A and passed through a slit (6), are pushed together out of the ring-form tip of an inner nozzle (1), and at the same time therewith, an outer film-forming liquid (5) for the outer soft capsule, as sent from B, is pushed out of the ring-form tip of an outer nozzle (2) arranged outside said ring-form tip and in the form of concentric circle, thereby to form a double jet which is then discharged into a cooling liquid (8).

In the prior art method wherein the filler is a liquid only, the encapsulation is made easy and the particle diameter is made uniform by giving moderate perturbation to the multiple jet flow using a perturbation means to cause the flow to be cut well. In the method of the present invention described above, however, the inner soft capsule (3), on being pushed out of the tip of the nozzle, acts to pull the outer capsule liquid (4) and outer film-forming liquid (5) into the flow, so that the same effect as with the perturbation means can be given to the multiple jet flow.

Of course, in the method of the present invention, a proper perturbation means may be employed if desired according to the kind and property of the inner soft capsule.

With the multiple soft capsule produced as above using the foregoing (a) and (b) as essential components for the film of the outer soft capsule, it suffices to use a vegetable oil, mineral oil or animal oil as a cooling liquid and after solidifying the capsule by cooling, to dry and wash the capsule as such.

While, with the double soft capsule produced as above using the foregoing (a), (b) and (c) as essential components for the film of the outer soft capsule, the capsule, after solidified by cooling, is brought into contact with a solution, preferably an aqueous solution of the salt of a polyvalent metal to gelatinize aqueous solutions of said lower methoxylpectin or sodium alginate. In this case, it is a common practice to dip the capsule to be treated in an aqueous solution containing 1 to 10 weight %, preferably 3 to 5 weight % of said salt at 5°C to 30°C, preferably 10°C to 15°C for 1 to 5 minutes, preferably 1 to 3 minutes. The contact between the double soft capsule and the treating liquid may be carried out, in addition to the dipping method, by other methods such as the spraying method. But the dipping method is the simplest as well as being applicable uniformly. The multiple soft capsule treated as above is then dried and washed.

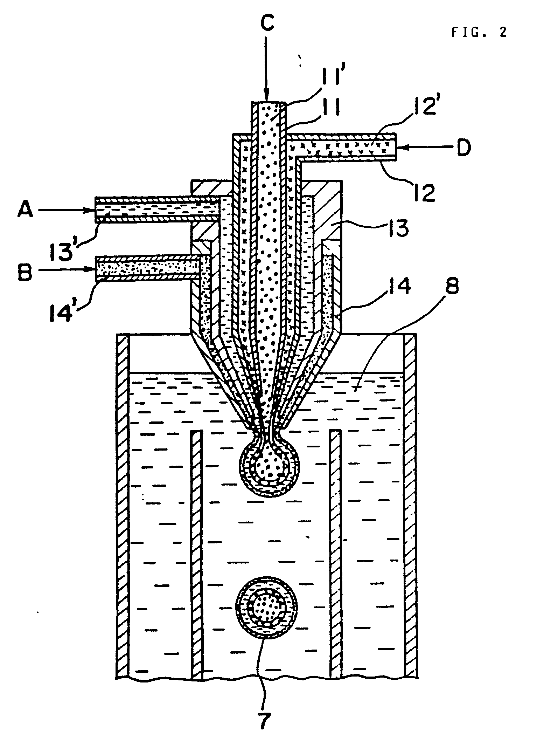

Another method suitable to produce the double soft capsule according to the present invention is a method wherein an inner capsule liquid, a film-forming liquid for the inner soft capsule (shortened as an inner film-forming liquid hereinafter), an outer capsule liquid and an outer film-forming liquid therefor are at the same time pushed out of the first, second, third and fourth nozzle respectively, as arranged in the form of concentric circles, having increasing radii in this order, thereby to form a multiple jet which is then discharged into a cooling liquid. Fig. 2 is a schematic vertical sectional view illustrating one embodiment of the nozzle part of an equipment for practicing this method.

In the equipment shown in Fig. 2, the double soft capsule (7) according to the present invention is obtained as follows:

An inner capsule liquid (11') sent from C, an inner film-forming liquid (12') sent from D, an outer capsule liquid (13') sent from A and outer film-forming liquid (14') sent from B are at the same time pushed out of the ring-form tips of the first (11), second (12), third (13) and fourth nozzle (14), respectively, as arranged in the form of concentric circles, having increasing radii in this order, thereby to form a multiple jet flow which is then discharged into a sealing liquid (8).

Also in this case, with the double soft capsule produced using the foregoing (a) and (b) as essential components for the film of the outer soft capsule, it suffices to dry and wash the capsule as such. But, with the multiple soft capsule produced using the foregoing (a), (b) and (c) as said components, the capsule, after having been treated as above, is dried and washed.

The present invention extends the use of soft encapsulated products in all the industrial fields, and particularly it is effective in combining two or more components which cannot be enclosed together in a single capsule because of incompatibility. Further, a synergistic effect and sustained effect can also be obtained depending upon the components contained.

The aesthetic appearance of the double soft capsule according to the present invention is expected to contribute to extending demands for said encapsulated product.

Next, the present invention will be illustrated with reference to the following examples.

Example 1

According to the conventional method, a garlic oil-containing inner soft capsule (particle diameter, 2 mm) was prepared using an inner film-forming liquid of the following composition.

Composition for the inner film-forming liquid:

|  | Parts by weight |
| --- | --- |
| Gelatin | 16 |
| D-sorbitol | 4 |
| Purified water | 80 |

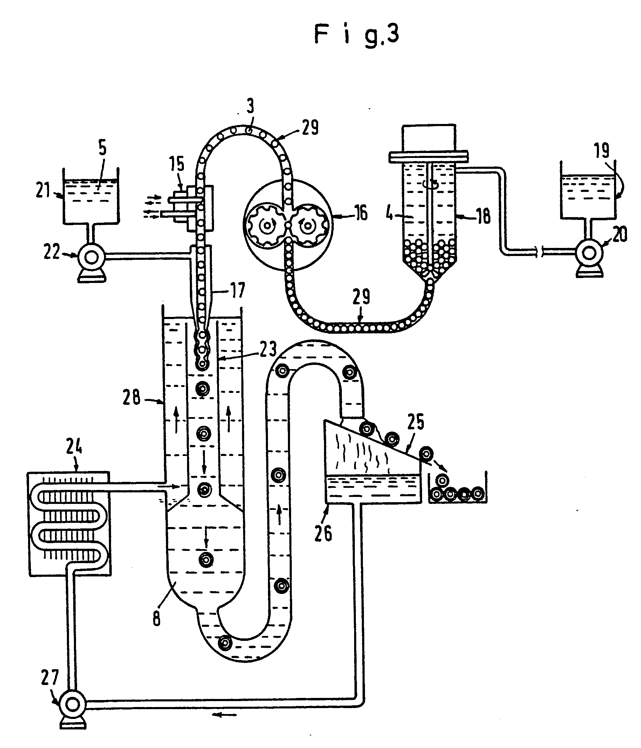

According to the flow sheet shown in Fig. 3, the inner soft capsule obtained above and a vitamin E-containing oil were encapsulated together using an outer film-forming liquid for outer soft capsule of the following composition to obtain a double capsule having an outside diameter of 6 mm.

Composition of the outer film-forming liquid:

|  | Parts by weight |
| --- | --- |
| Gelatin | 16 |
| D-sorbitol | 4 |
| Purified water | 80 |

The flow sheet in Fig. 3 will be illustrated later. A vitamin E-containing oil (4) (an outer capsule liquid) is sent from an outer capsule liquid storage tank (19) to an inner capsule storage tank (18) through a pump (20) to mix with inner soft capsules. The temperature of said outer capsule liquid (4) is kept constant (in this example, 20±1°C), and the viscosity of the liquid is preferably 400 mPa.s. The inner soft capsule (3) and the liquid (4) are sent together, at a flow rate of 0.3 ml/min., from the tank (18) through a pipe (29) (inside diameter, 2.5 mm), an extruder (16) and the pipe (29) to a double nozzle (17) (inner diameter, 3.0 mm; outer nozzle diameter, 6.0 mm). An aligning machine (15) is set between the extruder (16) and double nozzle (17) to keep the distance between the inner soft capsules constant (in this example, pitch number, 2.5 mm). The number of the inner soft capsules extruded is fixed at 8/sec. While an outer film-forming liquid (5), while being maintained at a constant temperature (in this example, 60±2°C), is sent, at a flow rate of 0.2 ml/sec, from a film-forming liquid storage tank (21) through a pump (22) to the double nozzle (17) to form a double jet which is then discharged into a cooling liquid (vegetable oil in this example). The cooling liquid (8) is kept at a low and constant temperature (9±0.5°C in this example) by a cooler (24) and circulated by means of a pump (27), at a flow rate of 240 ml/sec, through a cooling pipe (28) (inside diameter, 120 mm; length, 1000 mm), forming pipe (23) (inside diameter, 30 mm; length, 600 mm) set in the cooling pipe (28), tank (26), pump (27) and cooler (24) in this order. The capsules formed in the forming pipe (23) are sent, together with the circulating flow of the cooling liquid (8), to a separator (25) (sieve) wherein the capsules are separated from the cooling liquid (8), and then dried and washed. The outside diameter of the outer soft capsule of the double capsule thus obtained is 6±0.2 mm.

Example 2

In the same manner as in Example 1, a double capsule of the following composition was prepared.

Diameter of the inner soft capsule: 4 mm
Inner capsule liquid:

|  | parts by weight |
| --- | --- |
| ethyl cysteine hydrogen chloride | 30 |
| safflower oil | 70 |

Composition of an inner film-forming liquid:

|  | parts by weight |
| --- | --- |
| Gelatin | 75 |
| D-sorbitol | 20 |
| Sodium alginate | 5 |
| Water | 300 |

Diameter of the outer soft capsule: 8 mm
Outer capsule liquid:

|  | parts by weight |
| --- | --- |
| citric acid carbeta-pentane | 10 |
| safflower oil | 90 |

Composition an outer film-forming liquid:

| | parts by weight |
|---|---|
| Gelatin | 80 |
| D-sorbitol | 20 |
| Water | 350 |

Manufacturing condition:
    Pitch of aligning machine (15): 4.5 mm
    Forming pipe (23):  inside diameter, 30 mm;
                        length, 600 mm;
    Cooling pipe (28):  inside diameter, 120 mm
                        length, 1000 mm
    Pipe (29):  inside diameter, 4.5 mm
    Temperature of the outer film-forming liquid:
60°C
    Temperature of the outer capsule liquid: 20°C
    Temperature of the cooling liquid: 9°C
    Flow rate of the outer film-forming liquid:
0.4 ml/sec
    Flow rate of the outer capsule liquid:
0.3 ml/sec
    Flow rate of the cooling liquid: 240 ml/sec
    Number of inner capsules extruded: 8/sec

The outer soft capsule of the double capsule thus obtained is soluble in the stomach and the inner soft capsule thereof is soluble in the intestine, so that the efficacy of the respective medicines is displayed effectively.

## Claims

1. A double soft capsule wherein an inner soft capsule is contained with outer capsule liquid in an outer soft capsule, the film-forming substance of the inner and outer capsule comprising (a) gelatin and (b) a water-soluble polyhydric alcohol or its water-soluble derivative.

2. A double soft capsule according to Claim 1 which contains a medicine or a food stuff.

3. A double soft capsule according to Claim 1, wherein a film-forming substance for the inner and/or outer soft capsule is obtained by treating a composition containing (a) gelatin, (b) a water-soluble polyhydric alcohol or its water-soluble derivative and (c) a lower methoxylpectin having a molecular weight of not less than 200,000 and a degree of methoxylation of 1 to 6% or sodium alginate with a salt of a polyvalent metal to gelatinize an aqueous solution of said lower methoxylpectin or sodium alginate.

4. A double soft capsule according to Claim 1 or 3, wherein the water-soluble polyhydric alcohol is selected from glycerin and sorbitol.

5. A double soft capsule according to Claim 1, wherein the contents of gelatin and the water-soluble polyhydric alcohol or its water-soluble derivative are 60 to 90 weight % and 40 to 10 weight %, respectively, based on the total weight of the film.

6. A double soft capsule according to Claim 3, wherein the contents of gelatin, the lower methoxylpectin having a molecular weight of not less than 200,000 and a degree of methoxylation of 1 to 6% and the water-soluble polyhydric alcohol or its water-soluble derivative are 65 to 75 weight %, 5 to 20 weight % and 10 to 30 weight %, respectively, based on the total weight of the film.

7. A double soft capsule according to Claim 3, wherein the contents of gelatin, sodium alginate and the water-soluble polyhydric alcohol or its water-soluble derivative are 65 to 85 weight %, 1 to 10 weight % and 10 to 30 weight %, respectively, based on the total weight of the film.

8. A double soft capsule according to Claim 1, wherein the outer soft capsule is soluble in stomach and the inner soft capsule is soluble in intestine.

9. A method for producing a double soft capsule characterized in that a multiple jet, which is formed by pushing together (i) an inner soft capsule and (ii) an outer soft capsule liquid to be encapsulated in an outer soft capsule out of the ring-form tip of an inner nozzle and by pushing, at the same time therewith, (iii) an outer film-forming liquid for the outer soft capsule out of the ringform tip of an outer nozzle arranged outside said ringform tip and in the form of concentric circles is discharged into a cooling liquid.

10. A method for producing a double soft capsule according to Claim 9, wherein the outer film-forming liquid comprises (a) gelatin and (b) a water-soluble polyhydric alcohol or its water-soluble derivative.

11. A method for producing a double soft capsule according to Claim 9, wherein the outer film-forming liquid comprises (a) gelatin, (b) a water-soluble polyhydric alcohol or its water-soluble derivative and (c) a lower methoxylpectin having a molecular weight of not less than 200,000 and a degree of methoxylation of 1 to 6% or sodium alginate, and the obtained double soft capsule is further treated with a salt of a polyvalent metal to gelatinize an aqueous solution of said lower methoxylpectin or sodium alginate.

12. A method for producing a double soft capsule characterized in that a multiple jet, which is formed by pushing
    (i) an inner capsule liquid for an inner soft capsule,
    (ii) an inner film-forming liquid therefor,
    (iii) an outer capsule liquid for an outer soft capsule, and
    (iv) an outer film-forming liquid therefor, at the same time out of the first, second, third and fourth nozzle respectively, as arranged in the form of concentric circles, having increasing radii in this order, is discharged into a coating liquid.

13. A method for producing a double soft capsule according to Claim 12, wherein the inner and outer film-forming liquid comprises (a) gelatin and (b) water-soluble polyhydric alcohol or its water-soluble derivative.

14. A method for producing a double soft capsule according to Claim 12, wherein the outer film-forming liquid comprises (a) gelatin, (b) a water-soluble polyhydric alcohol or its water-soluble derivative and (c) a lower methoxylpectin having a molecular weight of not less than 200,000 and a degree of methoxylation of 1 to 6% or sodium alginate, and the obtained double soft capsule is further treated with a salt of polyvalent metal to gelatinize an aqueous solution of said lower methoxylation or sodium alginate.

**Patentansprüche**

1. Weiche Doppelkapsel, bei der eine weiche Innenkapsel zusammen mit einer Außenkapselflüssigkeit in einer weichen Außenkapsel enthalten ist, wobei die filmbildende Substanz der Innenkapsel und der Außenkapsel (a) Gelatine und (b) einen wasserlöslichen mehrwertigen Alkohol oder sein wasserlösliches Derivat enthält.

2. Weiche Doppelkapsel nach Anspruch 1, die ein Arzneimittel oder ein Lebensmittel enthält.

3. Weiche Doppelkapsel nach Anspruch 1, bei der eine filmbildende Substanz für die weiche Innenkapsel und/oder die weiche Außenkapsel erhalten wird, indem eine Mischung, die (a) Gelatine, (b) einen wasserlöslichen mehrwertigen Alkohol oder sein wasserlösliches Derivat und (c) ein niederes Methoxylpectin mit einem Molekulargewicht von nicht weniger als 200.000 und einem Methoxylierungsgrad von 1 bis 6% oder Natriumalginat enthält, mit einem Salz eines mehrwertigen Metalls behandelt wird, um eine wäßrige Lösung des niederen Methoxylpectins oder des Natriumalginats zu gelatinieren.

4. Weiche Doppelkapsel nach Anspruch 1 oder 3, bei der der wasserlösliche mehrwertige Alkohol aus Glycerin und Sorbit ausgewählt ist.

5. Weiche Doppelkapsel nach Anspruch 1, bei der der auf die Gesamtmasse des Films bezogene Gehalt der Gelatine und des wasserlöslichen mehrwertigen Alkohols oder seines wasserlöslichen Derivats 60 bis 90 Masse% bzw. 40 bis 10 Masse% beträgt.

6. Weiche Doppelkapsel nach Anspruch 3, bei der der auf die Gesamtmasse des Films bezogene Gehalt der Gelatine, des niederen Methoxylpectins mit einem Molekulargewicht von nicht weniger als 200.000 und einem Methoxylierungsgrad von 1 bis 6% und des wasserlöslichen mehrwertigen Alkohols oder seines wasserlöslichen Derivats 65 bis 75 Masse%, 5 bis 20 Masse% bzw. 10 bis 30 Masse% beträgt.

7. Weiche Doppelkapsel nach Anspruch 3, bei der der auf die Gesamtmasse des Films bezogene Gehalt der Gelatine, des Natriumalginats und des wasserlöslichen mehrwertigen Alkohols oder seines wasserlöslichen Derivats 65 bis 85 Masse%, 1 bis 10 Masse% bzw. 10 bis 30 Masse% beträgt.

8. Weiche Doppelkapsel nach Anspruch 1, bei der die weiche Außenkapsel im Magen löslich ist und die weiche Innenkapsel im Darm löslich ist.

9. Verfahren zur Herstellung einer weichen Doppelkapsel, dadurch gekennzeichnet, daß ein Mehrfachstrahl, der gebildet wird, indem (i) eine weiche Innenkapsel und (ii) eine Außenkapselflüssigkeit für eine weiche Außenkapsel, die in eine weiche Außenkapsel einzukapseln sind, zusammen aus der ringförmigen Mündung einer Innendüse ausgestoßen werden und indem gleichzeitig damit (iii) eine einen Außenfilm bildende Flüssigkeit für die weiche Außenkapsel aus der ringförmigen Mündung einer Außendüse, die außerhalb der erwähnten ringförmigen Mündung und in Form konzentrischer Kreise angeordnet ist, ausgestoßen wird, in eine Kühlflüssigkeit ausgelassen wird.

10. Verfahren zur Herstellung einer weichen Doppelkapsel nach Anspruch 9, bei dem die den Außenfilm bildende Flüssigkeit (a) Gelatine und (b) einen wasserlöslichen mehrwertigen Alkohol oder sein wasserlösliches Derivat enthält.

11. Verfahren zur Herstellung einer weichen Doppelkapsel nach Anspruch 9, bei dem die den Außenfilm bildende Flüssigkeit (a) Gelatine, (b) einen wasserlöslichen mehrwertigen Alkohol oder sein wasserlösliches Derivat und (c) ein niederes Methoxylpectin mit einem Molekulargewicht von nicht weniger als 200.000 und einem Methoxylierungsgrad von 1 bis 6% oder Natriumalginat enthält und bei dem die erhaltene weiche Doppelkapsel ferner mit einem Salz eines mehrwertigen Metalls behandelt wird, um eine wäßrige Lösung des niederen Methoxylpectins oder des Natriumalginats zu gelatinieren.

12. Verfahren zur Herstellung einer weichen Doppelkapsel, dadurch gekennzeichnet, daß ein Mehrfachstrahl, der gebildet wird, indem

(i) eine Innenkapselflüssigkeit für eine weiche Innenkapsel,

(ii) eine einen Innenfilm bildende Flüssigkeit dafür,

(iii) eine Außenkapselflüssigkeit für eine weiche Außenkapsel und

(iv) eine einen Außenfilm bildende Flüssigkeit dafür gleichzeitig aus einer ersten, einer zweiten, einer dritten bzw. einer vierten Düse, die in Form von konzentrischen Kreisen mit in dieser Reihenfolge zunehmenden Radien angeordnet sind, ausgestoßen werden, in eine Kühlflüssigkeit ausgelassen wird.

13. Verfahren zur Herstellung einer weichen Doppelkapsel nach Anspruch 12, bei dem die den Innenfilm bildende Flüssigkeit und die den Außenfilm bildende Flüssigkeit (a) Gelatine und (b) einen wasserlöslichen mehrwertigen Alkohol oder sein wasserlösliches Derivat enthalten.

14. Verfahren zur Herstellung einer weichen Doppelkapsel nach Anspruch 12, bei dem die den Außenfilm bildende Flüssigkeit (a) Gelatine (b) einen wasserlöslichen mehrwertigen Alkohol oder sein wasserlösliches Derivat und (c) ein niederes Methoxylpectin mit einem Molekulargewicht von nicht weniger als 200.000 und einem Methoxylierungsgrad von 1 bis 6% oder Natriumalginat enthält und bei dem die erhaltene weiche Doppelkapsel ferner mit einem Salz eines mehrwertigen Metalls behandelt wird, um eine wäßrige Lösung des niederen Methoxylpectins oder des Natriumalginats zu gelatinieren.

## Revendications

1. Capsule souple double dans laquelle une capsule souple interne est contenue avec un liquide de capsule externe dans une capsule souple externe, la substance constituant la pellicule des capsules interne et externe comportant (a) de la gélatine et (b) un alcool polyhydrique soluble dans l'eau ou son dérivé soluble dans l'eau.

2. Capsule souple double selon la revendication 1, qui contient un médicament ou un aliment.

3. Capsule souple double selon la revendication 1, dans laquelle une substance constituant une pellicule pour la capsule interne et/ou la capsule externe souple est obtenue en traitant une composition contenant (a) de la gélatine, (b) un alcool polyhydrique soluble dans l'eau ou son dérivé soluble dans l'eau et (c) une méthoxylpectine inférieure possédant un poids moléculaire supérieur à 200 000 et un degré de méthoxylation de 1 à 6% ou le l'alginate de sodium avec un sel d'un métal polyvalent pour gélatiniser une solution aqueuse de ladite méthoxylpectine inférieure ou de l'alginate de sodium.

4. Capsule souple double selon la revendication 1 ou 3, dans laquelle l'alcool polyhydrique soluble dans l'eau est choisi parmi la glycérine et le sorbitol.

5. Capsule souple double selon la revendication 1, dans lequel les teneurs en gélatine et en alcool polyhydrique soluble dans l'eau ou son dérivé soluble dans l'eau sont de 60 à 90% en poids et de 40 à 10% en poids, respectivement, par rapport au poids total de la pellicule.

6. Capsule souple double selon la revendication 3, dans laquelle les teneurs en gélatine, en méthoxylpectine inférieure possédant un poids moléculaire supérieur à 200 000 et un degré de méthoxylation de 1 à 6% et en alcool polyhydrique soluble dans l'eau ou son dérivé soluble dans l'eau sont de 65 à 75% en poids, 5 à 20% en poids et 10 à 30% en poids, respectivement, par rapport au poids total de la pellicule.

7. Capsule souple double selon la revendication 3, dans laquelle les teneurs en gélatine, en alginate de sodium et en alcool polyhydrique soluble dans l'eau ou son dérivé soluble dans l'eau sont de 65 à 85% en poids, 1 à 10% en poids et 10 à 30% en poids, respectivement, par rapport au poids total de la pellicule.

8. Capsule souple double selon la revendication 1, dans laquelle la capsule souple externe est soluble dans l'estomac et la capsule souple interne est soluble dans l'intestin.

9. Procédé de fabrication d'une capsule souple double caractérisé en ce qu'un jet multiple, qui est formé en poussant ensemble (i) une capsule souple interne et (ii) un liquide de capsule souple externe à encapsuler dans une capsule souple externe hors de l'extrémité en forme de bague d'une buse interne et en poussant, simultanément avec elle, (iii) un liquide formant une pellicule externe pour la capsule souple externe hors de l'extrémité annulaire d'une buse externe disposée à l'extérieur de ladite extrémité annulaire et sous la forme de cercles concentriques est déchargé dans un liquide de refroidissement.

10. Procédé de fabrication d'une capsule souple double selon la revendication 9, dans lequel le liquide formant la pellicule externe comporte (a) de la gélatine et (b) un alcool polyhydrique soluble dans l'eau ou son dérivé soluble dans l'eau.

11. Procédé de fabrication d'une capsule souple double selon la revendication 9, dans lequel le liquide formant la pellicule externe comporte (a) de la gélatine, (b) un alcool polyhydrique soluble dans l'eau ou son dérivé soluble dans l'eau et (c) une méthoxylpectine inférieure possédant un poids moléculaire supérieur à 200 000 et un degré de méthoxylation de 1 à 6% ou de l'alginate de sodium, de la capsule souple double obtenue est en outre traitée avec un sel d'un métal polyvalent pour gélatiniser une solution aqueuse de ladite méthoxylpectine inférieure ou de l'alginate de sodium.

12. Procédé de fabrication d'une capsule souple double caractérisé en ce qu'un jet multiple, qui est formé en poussant
   (i) un liquide de capsule interne pour une capsule souple interne,
   (ii) un liquide formant une pellicule interne à cet effet,
   (iii) un liquide de capsule externe pour une capsule souple externe, et
   (iv) un liquide formant une pellicule externe à cet effet, simultanément hors de la première, seconde, troisième et quatrième buses, respectivement, telles que disposées sous la forme de cercles concentriques, ayant des rayons croissants dans cet ordre, est déchargé dans un liquide de refroidissement.

13. Procédé de fabrication d'une capsule souple double selon la revendication 12, dans lequel le liquide formant la pellicule interne et externe comporte (a) de la gélatine et (b) un alcool polyhydrique soluble dans l'eau ou son dérivé soluble dans l'eau.

14. Procédé de fabrication d'une capsule souple double selon la revendication 12, dans lequel le liquide formant la pellicule externe comporte (a) de la gélatine, (b) un alcool polyhydrique soluble dans l'eau ou son dérivé soluble dans l'eau et (c) une méthoxylpectine inférieure possédant un poids moléculaire supérieur à 200 000 et un degré de méthoxylation de 1 à 6% ou de l'alginate de sodium, et la capsule souple double obtenue est en outre traitée avec un sel d'un métal polyvalent pour gélatiniser une solution aqueuse de ladite méthoxylpectine inférieure ou de l'alginate de sodium.

FIG. 1

FIG. 2

EP 0 116 311 B1

Fig.3

Fig.4